# EUROPEAN PATENT SPECIFICATION

(11) **EP 0 825 877 B1**
(45) Date of publication and mention of the grant of the patent: **22.12.2004**
(21) Application number: 96914498.9
(22) Date of filing: 03.05.1996
(51) Int. Cl.: A61K 47/48, A61K 31/714, A61P 27/12

(54) **COMPOSITION FOR PREVENTION OF POSTERIOR CAPSULE OPACIFICATION**
MITTEL ZUR VERHINDERUNG DER TRUEBUNG DER VORDEREN LINSENKAPSEL
COMPOSITION PREVENANT L'OPACIFICATION DE LA CAPSULE POSTERIEURE

(30) Priority: 05.05.1995 SE 9501671
(43) Date of publication of application: 04.03.1998
(73) Proprietor: Pfizer Health AB, 112 87 Stockholm (SE)
(72) Inventor: EDSMAN, Katarina, S-757 56 Uppsala (SE); GÖLANDER, Carl-Gustaf, S-756 53 Uppsala (SE); WICKSTRÖM, Kerstin, S-752 33 Uppsala (SE)
(74) Representative: Rosenquist, Per Olof
(86) International application number: PCT/SE1996/000588
(87) International publication number: WO 1996/034629

(56) References cited:
- EP-A- 0 392 487
- STN INTERNATIONAL, File CAPLUS, CAPLUS accession No. 1992:439939, CERA C. et al., "Water-Soluble Polysaccharide-Anthracycline Conjugates: Biological Activity"; & ANTI-CANCER DRUG DES., (1992), 7(2), 143-51.
- DIALOG INFORMATION SERVICES, File 155, MEDLINE, Dialog Accession No. 06852860, MEDLINE Accession No. 89154860, WELLER M. et al., "Evaluation of Daunomycin Toxicity on Lens Epithelium In Vitro"; & INT. OPHTHALMOL., (NETHERLANDS), 1988, 12(2), p. 127-30.
- DIALOG INFORMATION SERVICES, File 155, MEDLINE, Dialog Accession No. 07693079, MEDLINE Accession No. 91212079, RUIZ J.M. et al., "Inhibition of Posterior Capsule Opacification by 5-Fluorouracil in Rabbits"; & OPHTHALMIC. RES., (SWITZERLAND), 1990, 22(4), p. 201-8.

## Description

The present invention is related to the use of well defined controlled release systems, for instance complexes between hyaluronic acid and cytotoxic agents like Doxorubicin, for prevention of undesired side-effects after intraocular lens implantation.

Posterior Capsule Opacification (PCO), also named after-cataract or "secondary cataract" is the only major complication following cataract surgery. It is caused by proliferation and migration of remnant lens epithelial cells on the anterior capsule wall and occurs as localized assemblies of cells (Elschnigs' pearls) and/or as fibrosis (myofibroblasts) on larger areas of the posterior capsule ( D J Apple et al, Survey of Ophthalmologie, 37:2 (1992), 73-116 and E P Steinberg et al; Arch Ophthalmol, 111 (1993), 1041-1049)

Clinical incidences between 10 and 50 % has been reported with follow-up times of 1 to 5 years. A recurrent figure is 30 % within 2 years and higher figures are reported for younger people and Asians. It is also a partially hidden problem since many elderly patients do not seek a doctor until vision almost has disappeared.

The standard treatment today is YAG-laser capsulotomy ("YAG") of the posterior capsule. Even though YAG usually is successful, higher incidences of side-reactions such as retinal detachment, cystoid macular oedema, uveitis and lens damage has been reported. Furthermore, YAG constitutes a rather expensive method of treatment from a social economy point of view and requires at least two visits to the doctor. It is also important to consider that the patient is suffering from a disturbed vision for a certain time prior to the YAG treatment and that in many developing countries where YAG is not available, PCO is a common cause of blindness.

Hence, from a quality of life view point, an alternative method for treatment or preferably prevention would be desirable and solutions have been sought along three different lines: IOL design, surgical techniques and pharmacological inhibition.

Biconvex IOLs with angulated loops and plate lens designs has been developed with the aim to press against the posterior capsule and to avoid PCO according to the concept "no space-no cells". An alternative lens design (Hoffer, US Patent 4244060) contains a ridge extending rearwardly from the rear surface of the lens body and which seats against the capsule wall and thereby prevents cells from migrating into the space behind the optics. Surface modified intraocular lenses and the use of marine mussel glue to adhere the IOL to the capsule wall has also been tried. The reported effect on PCO is very limited. The development of full size injectable lenses with a similar objective is still in it's infancy.

Various surgical techniques and aids has been reported such as better polishing techniques, hydrodissection, cell-lysis by sterile water and other hypotonic solutions, peptidising aids e.g. EDTA/trypsin, cryoapplication and UV-radiation etc. None of these methods has been reported to be successful in clinical tests.

Antimetabolites has traditionally been used for preventing cell-growth and wound healing in patients with risk for failure in glaucoma filtering surgery (trabeculectomy). A subconjunctival administration of 5-fluorouracil (5-FU) or Mitomycin C inhibits fibroblast proliferation stimulated by filtering surgery The therapeutic dose is often close to its toxic level which, however, is rationalized by the high risk of blindness for glaucoma patients. Other cytotoxic agents that have been evaluated for inhibitory potential on eye tissues are Doxorubicin, Daunomycin, Vincristine, Cisplatin, Bleomycin sulfate, Etoposide, cytarabine, colchicine, thiotepa etc (Peyman GA et al, Ophthalmic Surgery 15:5 (1984) 411-413).

Experiments are now ongoing to use cytotoxic agents to prevent PCO. The rationale is to use the natural targetting effect of antimetabolites to rapidly dividing mitotic lens epithelial cells while avoiding toxic side-reactions on non-mitotic cells such as corneal endothelial cells, iris pigment epithelial cells and retinal cells. The drug is injected in the anterior chamber or in the lens capsule in conjunction with cataract surgery. Antimetabolites that has been studied in single dose administration include 5-FU, colchicine, retinoic acid, methotrexate, daunomycin, doxorubicin, cytosine arabinose (see for instance MacDonnell et al, Ophthalmic Surgery 19:1 (1988) 25-30 and Hartmann P et al Ophthalmologie 4 (1990) 102-106). Daunomycin has also been shown to reduce PCO in clinics. In order to improve the targeting effect of theses drugs, a number of antibody-antimetabolite conjugates (where the antibody is directed against lens epithelial cell membrane or to collagen in the lens capsule) have been disclosed, such as a complement fixing mab, ricin A-mab conjugate and methotrexate-mab conjugate. Fibroblast growth factor (FGF) has also been utilized as a targetting moiety (FGF receptors on lens epithelial cells) in a conjugate with saporin (cytotoxic). Single dose administration of growth factor inhibitors (Lens epithelial necrosis factor) was tested by Hunold (Fortschr Ophthalmol 88:4 (1989) 386-389).

A drawback with single dose administration is that relatively large amounts of cytotoxic drugs is required in order to achieve significant reduction of PCO which may cause local toxic reactions in the eye tissue Roy FH and Olmos E, Contact and Intraocular Lens Med J 5: (1979) 175-178). Also rather high dosages results in only limited reduction in PCO the reason being that the biological half-life of the drug in the aqueous humor is relatively short (hours) in relation to the the lens epithelial cell division cycle.

Sustained release of cytotoxic agents in the eye to avoid toxic drug levels while maintaining a constant therapeutic concentration over a prolonged period of time was first used to improve the success rate in glaucoma filtering surgery. Examples of such systems are 5-FU loaded into ethyl-vinyl acetate copolymer membranes or biodegradible or bioerodible erodible copolymers of poly(lactide)-poly(glycolide) (PLG), copolymers, polyorthoesters (POE), poly(caprolactam) (PCL), and Mitomycin C, Etopiside and Taxol in polyanhydrides (PAH). Doxorubicin in PLG copolymers has been used for drug release in the vitreous against proliferative vitreoretinopathy. Generally, drug release ranging up to more than several months was obtained with these systems and with good efficacy.

Similarly, sustained delivery systems has been tested against PCO. Solomon et al (Invest Ophthalmol & Visual Sci (suppl) 31 (1990) 351) have shown that sustained release of 5-FU in vivo in a rabbit model can maintain a therapeutic level over a prolonged time period leading to a significant reduction in PCO compared to control. A similar result was obtained by Legler et al using colchicine encapsulated in a diffusion limiting membrane (J Cataract Refract Srug 19 (1993) 460-470). Another interesting delivery system is the IOL itself e.g. a heparin-surface modified IOL for slowly releasing FGF-Saporin. In these systems drug release times between 1 week and 40 days was obtained.

Also sustained delivery systems suffer from certain draw-backs. Most of the cytotoxic drugs are cancerogenic and/or teratogenic and this risk increases with exposure time. Long-term effects on the iris ciliary muscle and retinal function (changes in electroretinogram, ERG) has also been reported. Tetz (Docent thesis:Die Cataracta secundaria nach Hinterkammarlinsenimplantation: Klinik, Pathologie und Möglichkeiten der Prävention, Augenklinik der Universität Heidelberg, Januar 1994.) found that 1 µg/day delivered up to 6 months resulted in complete inhibition of PCO but also to severe local toxic effects in the eye. Furthermore, the bioerodible vehicles may induce an inflammatory response which may itself trigger proliferation.

Considering these effects there is a need to reduce the exposure time to the cyotoxic drug to a minimum..

In order to adapt the drug release time to the cell division cycle of lens epithelial cells and possible to reduce the effective delivery time to a minimum, the lens epithelial cell division after lens extraction was studied in rabbits and monkeys. Cells entering the S-phase of the cell cycle, preparing for cell division, actively take up BrdU. This was used to quantify cell division with immunochemical methods. At various times after lens extraction animals were injected with BrdU prior to killing. As will be demonstrated in Example 1 below, the relative number of BrdU positive rabbit LEC nuclei peaked at day 1-2 with 30 and 20% labelled cells in the untreated eyes. The percentage of labelled cells declined to 1% at day 7 and stayed at this low level throughout the two-month study. Monkey LEC had a lower rate of proliferation and also a later onset with a maximal BrdU incorporation 5 days after surgery.

Accordingly, rabbit LEC proliferation is an early event with most of the cells in S-phase already the first days after surgery. However, in the primate eye where LEC proliferation is longer in duration and slower in onset, a slow release delivery of an inhibitory compound is necessary to fully prevent PCO.

Very good correlation was obtained with in vivo data, as will be further demonstrated.. Accordingly, in a rabbit model, where PCO was measured as wet mass, 5 mg of 5-FU administered as a single dose resulted in 50 % inhibition of PCO, the same amount delivered over 2-3 days resulted in 70% inhibition and the same amount delivered over10-12 days resulted in > 90 % inhibition.

The invention is accordingly related to a method for prevention of PCO in primates, especiall man, comprising administration into the eye of a controlled release composition which is characteristed by releasing more than 30%, preferrably more than 40% and especially more than 50% of the total amount of the drug during day 1-7, e.g. day 1-5 and day 1-3 after surgery and to contain less than 10%, preferrably less than 5% and especially less than 1% of the drug at day 14 and esp. day 10 after surgery.

These relatively short delivery times can be obtained for example by forming a sparingly soluble complex between the cytotoxic drug and a vehicle. For anthracyclines like Doxorubicin, Daunorubicin, Epirubicin etc which are cationic and relatively hydrophobic drugs, such complexes can be formed with polyanions like hyaluronic acid. Hyaluronic acid in uncomplexed, complexed or conjugate form (prodrug) has been used as a drug vehicle to improve bioavailability e.g. penetration of the drug into cell tissues (Falk et al WO Patent Publication 9104058). Daunorubicin and Doxorubicin has also been covalently linked to the carboxyl group of hyaluronic acid and other carboxylated polysaccharides for transporting the drug to the tumor site (Hamana WO Patent Publication 9419376) or blended into a biodegradable polymer of PLG copolymer with hyaluronic acid etc in order to obtain slow release of the drug (Canal et al EP 486959).

In contrast to this, we have utilized the reduced diffusion properties of hyaluronic acid (HA)-complexed cationic cytotoxic drugs in order to maintain concentration over the range of 1-10, preferrably 1-7 days, which we have found critical for the anti-PCO effect. The diffusion rate can be controlled by controlling the molar ratio CT drug/HA. It is important to note that hyaluronic acid itself has no effect on the bioavailability of the drug since formulations with drugs which do not complex HA e.g. 5-FU-HA has no advantage compared to 5-FU in saline in terms of efficacy against PCO.

The cationic cytotoxic drug (for example Doxorubicin) in an acidic water solution is mixed with 0.2 - 5 %, for instance around 1%, hyaluronic acid under thorough mixing. In order to avoid precipitation, the cytotoxic drug is preferrably added in less than stoechiometric amounts, even if precipitates potentially can be utilised. 10-100 µl of this mixture is injected into the capsular bag after extracapsular cataract extraction (ECCE) and implantation of a capsular lens. The lump is applied between the IOL and the posterior capsule, preferably in contact with the posterior capsule and close to the equator and is left in the eye. Such small amounts of HA does not result in elevated IOPs which otherwise has been noticed when Healon is left in the anterior capsule after cataract surgery. The viscous CTdrug-HA lump preferrably attaches to the capsule or the posterior side of the IOL to avoid clearance out of the eye with the convective flow of aqueous humor which is 2-3µl/h in humans and 3-5 µl/h in rabbits. By varying the CT drug/HA molar ratio, various release times by diffusion can be obtained. If a stoechiometric complex (precipitate) is formed, the diffusion rate is very slow and amounts to several days. This is because hydrophobic pockets of CT drug are formed within the complex which are less available to dissolution by water.

In one embodiment of the invention a complex comprising a high viscosity compound like HA or other carboxylated or sulfated polysaccharide anions and a cationic drug, preferable a cytotoxic one, e.g. belonging to the groups of anthracyclines, like Mitomycin etc, is utilized. A key characteristic of the complex is the controlled release of the drug by slow diffusion into the lens capsule during up to 10 days and which results in a significantly larger reduction of PCO (> 60 %) compared to the corresponding free form of the cytotoxic drug would give at the same concentration.

The examples of systems that give sustained release and have the advantage of a high viscosity for a slower mixing with aqueous humor are physical gels. There are many gels and high viscosity polymers that can be used for sustained release of the active compound to fit the release profile described. The release may depend on specific interations between the polymer chain as in the example with doxorubicin and hyaluronate. The release may also be sustained as a result of nonspecific interations and for some active components, especially components of larger sizes, the release will be hindered.
Lipid systems show a wide variety of physical forms and release principle; they are solid, semisolid and liquid lipid systems. The most commonly used are the liquid systems that can give a sustained release. The liposomes are further advantageous since they are known to decrease the toxicity of the drug (Maisner et al, Adv Drug Delivery Reviews 16 (1995) 75-93. For further examples, see Cavalli et al, Int J Pharm 89 (1993) R9-R12).

The examples of systems for delivery of the active compound during a specified delivery time comprises polyanhydrides, see for instance Inv Ophthalmol & Vis Sci 29(11) (1988) 1692-7 in which 5-Fu was delivered over one week from polyanhydride. Other examples are polyorthoesters, polycaprolactone and PLG (for a review see Okada et al, Critical Revies in Ther. Drug Carrier Systems 12(1) (1995) 1-99), Wada et al, Bull Inst Chem Res, Kyoto Univ 66(3) (1988) 241-250, Seymor et al, J Controlled Release, 31 (1994) 201-206 and Merkli et al, J Controlled Release, 33 (1995) 415-421).

The patent application will now be illustrated by the following examples.

### Example 1. Cell division in the ocular lens of rabbits and monkeys after lens extraction.

Extracapsular lens extraction was performed in NZW rabbits and *Macaca fascicularis* monkeys. Prior to surgery, the pupil dilatation was performed by repeated topical application of Cyclogyl® 1 % (Alcon) and Neosynephrine® 10 % (Sterling Winthrop, WY, USA) twice with approximately 5 minutes interval. The animals were anaesthetised with Ketalar® (Parke-Davies, UK), 35 mg/kg and Rompun® (Bayer AG, FRG) 5 mg/kg, i.m. Topical Tetracain® (Alcon, Texas, USA) was applied for local anaesthesia. At various times from 8 hours to 56 days after surgery, the rabbits received an i.p. injection of 3 mg/kg BrdU (ZYMED, San Francisco, USA). After 2 more hours, the animals were killed with an overdose of pentobarbiturate and the eyes were enucleated.

The number of monkey LEC in S-phase were evaluated in 12 animals at 0, 1, 3, 5, 8 and 13 days after lens extraction and these animals were sacrificed three hours after BrdU injection. All eyes were prepared for immunohistochemical analysis of S-phase incorporated BrdU.

After enucleation, the eyes were fixed in formalin and routinely processed for paraffin embedding. Sections of 3-4 µm were cut at the pupillary opening, deparaffinised, rehydrated and stained for BrdU according to the ZYMED BrdU Staining Kit (ZYMED, San Francisco, USA).

The total number of LEC were counted in one section at comparable levels at the pupillary opening and the percentage of BrdU positive nuclei was determined.

Data are reported as a group mean ± S.D. The groups were compared over time with one-way ANOVA. Further evaluation was performed with Tukey's test. p < 0.05 was considered significant.

Normal LEC from control rabbits without lens extraction had a low proliferative rate. In most of these eyes, no cells were in S-phase and the maximal BrdU-labelling was 0.5 %. Approximately the same figures were observed at 10 and 14 hours after lens extraction. The percentage of positive cells in the untreated eyes peaked at day 1 and stayed high at day 2 with 30 and 20 % labelled cells respectively. Thereafter, a rapid decline in S-phase cells was seen. At day 7 and throughout the 2 months study, the level was close to that in the control LEC

**Table 1.**

| Rabbits | | | Monekys | |
|---|---|---|---|---|
| Day | %BrdU | S.D. | %BrdU | S.D. |
| 0 | 0.13 | 0.18 | 0 | 0 |
| 0.42 | 0 | 0 | | |
| 0.58 | 0.2 | 0.35 | | |
| 1 | 32.3 | 17.48 | 0 | 0 |
| 2 | 19.32 | 9.94 | | |
| 3 | 6.73 | 3.33 | 0.46 | 0.38 |
| 4. | 2.36 | 1.43 | | |
| 5. | 1.48 | 0.39 | 2.91 | 2.23 |
| 6 | 1.31 | 0.36 | | |
| 7 | 1.1 | 0.54 | | |
| 8 | | | 1.35 | 0.97 |
| 13 | | | 1.28 | 0.48 |
| 14 | 1.1 | 0.53 | | |
| 28 | 1.4 | 0.84 | | |
| 56 | 0.34 | 0.35 | | |

The relative number of BrdU positive rabbit LEC nuclei peaked at day 1-2 with 30 and 20 % labelled cells in the untreated eyes. The percentage of labelled cells declined to 1 % at day 7, and stayed at this low level throughout the two-month study. Monkey LEC had a lower rate of proliferation and also a later onset with a maximal BrdU incorporation 5 days after surgery.

Rabbit LEC proliferation is an early event with most of the cells in S-phase already the first days after surgery. However, in the primate eye where LEC proliferation is longer in duration and slower in onset, a slow release delivery of an inhibitory compound might be necessary to fully prevent PCO.

### Example 2: Doxorubicine in Hyaluronate.

A solution of 2 mg/ml of doxorubicine at low pH was mixed with hyaluronate solution giving a total dose of doxorubicine between 0.1 to 10 µg when applied in a total volume of about 10- 100 ul. The concentration of hyaluronate in the final mixture is preferrably between 2 and 50 mg/ml. The final formulation is administered after cataract surgery through a cannula, in the space between the intraocular lens and the posterior lenscapsule before closing the eye.

Doxorubicin is slowly released from the hyaluronate-doxorubicine solution. The diffusion of doxorubicine in hyaluronate was found to be much lower than in saline solution due to complex formation between the negatively charged hyaluronate and the positively charged doxorubicine. The diffusion of Doxorubicine out of the hyaluronate solution is more than three times slower than the diffusion of doxorubicine in water. In a diffusion experiment using 100 µg/ml of Doxorubicin in a Sundelöfcell (Sundelöf L-O, 1982, Anal. Biochem, 127,282-286, Laurent T C, Preston B N Sundelöf L-O Van Damme M P, 1982, Anal. Biochem, 127, 287-292), it took 20 hours for 20 % of doxorubicin in water to diffuse into the upper compartment as compared to 70 hours for the doxorubicine in hyaluronate (10 mg/ml).

The combination of the slow diffusion and the high viscosity of hyaluronate will result in a much longer contact time between the active component doxorubicin and the remaining lens epithelial cells. A saline solution of doxorubicin will instantanously mix with the aqueous humor but the hyaluronate solution will stay between the IOL and the posterior lens capsule for a longer time.

### In vivo studies in rabbits

Albino rabbits were used. In both eyes the crystalline lens was removed by phacoemusification. In both eyes a PMMA IOL was implanted into the the capsular bag. In one eye 100 µl of the doxorubicin-Healon slution was injected into the capsular bag behind the IOL. The fellow eye received 100 µ 1 Healon. Two months postoperatively the animals were killed, and the secondary cataracts were removed and weighed to the neariest mg. In one group the doxorubicin was injected dissolved in ta buffer solution instead of Healon. This in order to compare the efficacy when doxorubicin was dissolved in either Healon or buffer.
There was a dose dependent inhibitory effect of doxorubicin dissolved in Healon. The doses of 3, 1 and 0.3 µg all gave a significant decrease of the secondary cataract formation. When comparing the dosage 1 µg injected in either Healon of buffer, there was a significant better inibitory effect when doxorubicin was delivered in Healon.

### The amount of secondary cataract tissue was typically:

| | |
|---|---|
| control in buffer | 74 ±25 mg |
| control in Healon | 74 ±22 mg |
| 1 µg doxorubicin in buffer | 65 ±15 mg |
| 1 µg doxorubicin in Healon | 38 ±15 mg |

### Example 3: Slow release of 5-fluoro-uracil (5-FU).

5 mg 5-FU was put in a matrix comprising an Eudragit layer on the intraocular lens (IOL). Additional coatings with Eudragit were made in order to get a constant release for the predetermined time, the invivo release of 5-FU was 3 (SR-3) or 12 (SR-12)days. The release rate from the lenses were determined in vitro and in vivo (for invitro-invivo correlation). The in vitro release studies from the lens were made at 37 °C. The in vivo release studies were made on albino rabbits (New Zealand White), the IOL's containing 5-FU were implanted into the lens capsule after extracapsular lens extraction. The lenses were explanted at different times after implantation and the concentration of 5-FU in the aqueous humour and also the amount of 5-FU remaining on the lens was determined (Tables 1 and 2). The in vitro release from the lenses was relatively constant over the releasing period.,The release from SR-3 were 0.60-0.80 mg/day for 7 days and for SR-12 the relase was 0.20-0.35 mg/day for 21 days.. The in vivo release rate was approximately twice the rate in vitro.

**Table 1.**

| Time after implantation of SR-3 (days) | Amount remaining on lens (mg) | Conc. of 5-FU in aq. humour (µg/ml) |
|---|---|---|
| 0 | 5-5.5 | |
| 1 | 3.2-4.6 | 84 |
| 1.5 | 2.9 | 64 |
| 2.5 | 0.04 | 17 |

**Table 2.**

| Time after implantation of SR-12 (days) | Amount remaining on lens (mg) (4 lenses) |
|---|---|
| 2 | 3.3-3.5 |
| 4 | 2.0-2.6 |
| 6 | 1.7-2.3 |
| 8 | 1.2-2.0 |
| 10 | 0.8-1.2 |
| 12 | 0.05-0.3 |
| 14 | 0.00-0.3 |

### Example 3.In vivo results of single dose compared to slow release (SR).

5 mg of 5-FU was given to rabbits either as a single dose (instant release) or as a slow release formulation prepared according to Example 3 where 5 mg 5-FU was released over 3 or 12 days in vivo. The amount of secondary cataract formation in the rabbits were evaluated according to Lundgren at al (Lundgren B, Jonsson E and Rolfsen W, 1992, Acta Ophthalmol. Suppl 205:25-28). The single saline dose of 5-FU reduced the secondary cataract formation by 50% while the SR-formulation (SR-3 and SR-10) reduced the secondary cataract formation by 70 and 100% respectively.

### Example 5: Slow release of doxorubicin

A solution of doxorubicin and polyethylene glycol was mixed with a dispersion of Eudragit giving a concentration of doxorubicin of 1mg/ml. The mixture was pipetted onto an intraocular lens (IOL) and were allowed to dry for a few days. The total dose of doxorubicin can be varied by varying the volume pipetted onto the lens. In vitro release studies from the lens were made at 37 °c (Table 3). From Example 3 it is known that the in vivo release is appoximately twice as fast as the in vitro releaase in the system used. This formulation gives a relatively constant release in vivo over 10-12 days.

**Table 3**

| Time (days) | Released amount of doxorubicin (%) |
|---|---|
| 2 | 15 |
| 4 | 30 |
| 6 | 42 |
| 8 | 50 |
| 10 | 61 |
| 12 | 71 |
| 14 | 80 |
| 16 | 86 |
| 18 | 92 |
| 20 | 99 |

## Claims

1. An ophthalmological composition for preventing posterior capsule opacification, comprising a cationic cytotoxic agent and a carrier, **characterized in that** said carrier is a negatively charged polymer and said composition, which is a controlled release composition, releases more than 30% of the total amount of said cationic cytotoxic agent during day 1-7 after surgery and contains less than 10% of said cationic cytotoxic agent at day 14 after surgery.

2. A composition according to claim 1, wherein said polymer is hyaluronic acid.

3. A composition according to claim 1 or 2, wherein said cationic cytotoxic agent forms a complex with the carrier.

4. A composition according to any one of claims 1-3, wherein said cationic cytotoxic agent is an anthracycline.

5. A composition according to claim 4, wherein said anthracycline is selected from the group consisting of doxorubicin and daunorubicin.

6. A composition according to any one of claims 2-5, wherein the concentration of hyaluronic acid in said composition is in the range of 0.2-5%.

7. A composition according to claim 6, wherein the concentration of hyaluronic acid in said composition is around 1%.

8. Use of a composition according to any one of claims 1-7 for the manufacture of a medicament for prevention of posterior capsule opacification.

9. Use of a composition according to any one of claims 1-7 for the manufacture of a medicament for injection into the lens capsule in conjunction with cataract surgery.

## Patentansprüche

1. Ophthalmologische Zusammensetzung zur Verhütung einer Trübung der hinteren Augenkapsel, umfassend ein kationisches zytotoxisches Mittel und einen Träger, **dadurch gekennzeichnet, dass** der Träger ein negativ geladenes Polymer ist und die Zusammensetzung, die eine Zusammensetzung mit gesteuerter Freisetzung ist, mehr als 30 % der Gesamtmenge des kationischen zytotoxischen Mittels während des 1. bis 7. Tages nach der Operation freisetzt und am 14. Tag nach der Operation weniger als 10 % des kationischen zytotoischen Mittels enthält.

2. Zusammensetzung nach Anspruch 1, wobei das Polymer Hyaluronsäure ist.

3. Zusammensetzung nach Anspruch 1 oder 2, wobei das kationische zytotoxische Mittel einen Komplex mit dem Träger bildet.

4. Zusammensetzung nach einem der Ansprüche 1 bis 3, wobei das kationische zytotoxische Mittel ein Anthracyclin ist.

5. Zusammensetzung nach Anspruch 4, wobei das Anthracyclin ausgewählt ist aus der Gruppe, bestehend aus Doxorubicin und Daunorubicin.

6. Zusammensetzung nach einem der Ansprüche 2 bis 5, wobei die Konzentration der Hyaluronsäure in der Zusammensetzung im Bereich von 0,2 bis 5 % liegt.

7. Zusammensetzung nach Anspruch 6, wobei die Konzentration der Hyaluronsäure in der Zusammensetzung etwa 1 % beträgt.

8. Verwendung einer Zusammensetzung nach einem der Ansprüche 1 bis 7 zur Herstellung eines Arzneimittels zur Verhütung einer Trübung der hinteren Augenkapsel.

9. Verwendung einer Zusammensetzung nach einem der Ansprüche 1 bis 7 zur Herstellung eines Arzneimittels zur Injektion in die Linsenkapsel im Zusammenhang mit einer Staroperation.

## Revendications

1. Composition ophtalmologique pour prévenir l'opacification de la capsule postérieure, comprenant un agent cytotoxique cationique et un support,
**caractérisée en ce que** ledit support est un polymère chargé négativement et ladite composition, qui est une composition à libération contrôlée, libère plus de 30% de la quantité totale dudit agent cytotoxique cationique au jour 1-7 après l'intervention chirurgicale et contient moins de 10% dudit agent cytotoxique cationique au jour 14 après l'intervention chirurgicale.

2. Composition selon la revendication 1, dans laquelle ledit polymère est l'acide hyaluronique.

3. Composition selon la revendication 1 ou 2, dans laquelle ledit agent cytotoxique cationique forme un complexe avec le support.

4. Composition selon l'une quelconque des revendications 1-3, dans laquelle ledit agent cytotoxique cationique est une anthracycline.

5. Composition selon la revendication 4, dans laquelle ladite anthracycline est choisie dans le groupe consistant en doxorubicine et daunorulicine.

6. Composition selon l'une quelconque des revendications 2-5, dans laquelle la concentration d'acide hyaluronique dans ladite composition est dans la plage de 0,2-5%.

7. Composition selon la revendication 6, dans laquelle la concentration d'acide hyaluronique dans ladite composition est d'environ 1%.

8. Utilisation d'une composition selon l'une quelconque des revendications 1-7 pour la fabrication d'un médicament destiné à la prévention de l'opacification de la capsule postérieure.

9. Utilisation d'une composition selon l'une quelconque des revendications 1-7 pour la fabrication d'un médicament destiné à l'injection dans le cristalloïde en liaison avec une intervention chirurgicale de la cataracte.
